# EUROPÄISCHE PATENTANMELDUNG

(11) **EP 1 306 064 A1**
(43) Veröffentlichungstag der Anmeldung: **02.05.2003**
(21) Anmeldenummer: 01125793.8
(22) Anmeldetag: 29.10.2001
(51) Int. Cl.: A61F 2/46

(54) **Instrument zum Einsetzen einer Zwischenwirbelprothese**

(71) Anmelder: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(57) **Zusammenfassung**

Instrument zum Einsetzen einer Zwischenwirbelprothese, das zwei durch eine Parallelführung (56,57) verbundene, voneinander wegspreizbare Prothesenhalterungen (52) für ein Paar von Prothesenplatten aufweist. Die erste Prothesenhalterung (52A) ist fest an einem langgestreckten Instrumentenkörper (51) angeordnet. Die zweite Prothesenhalterung (52B) ist vermittelst einer Parallelführung von dem Instrumentenkörper (51) gehalten. Es ist ein Schräglenker (57) vorgesehen, dessen erstes Ende (58) an dem Instrumentenkörper (51) in dessen Längsrichtung beweglich und dessen zweites Ende (60) an dem zweiten Prothesenhalter (52B) festachsig angelenkt ist. Das erste Ende (58) des Schräglenkers (57) ist mit einer in Längsrichtung des Instrumentenkörpers (51) bewegbaren Betätigungseinrichtung verbunden.

## Beschreibung

Zum Einsetzen von Zwischenwirbelprothesen, die aus zwei jeweils mit einem Wirbelkörper zu verbindenden Prothesenplatten und einem dazwischen angeordneten Prothesenkern bestehen, sind Einsetzinstrumente bekannt (EP-A-333 990), die an ihrem vorderen Ende zwei Prothesenhalterungen aufweisen, die jeweils eine Prothesenplatte aufnehmen. Die Prothesenhalterungen sind durch eine Parallelführung miteinander verbunden, die es gestattet, die Prothesenplatten zunächst einander stark anzunähern, um sie leichter in den engen Zwischenwirbelraum einbringen zu können, und anschließend (mit den angrenzenden Wirbeln) auseinander zu spreizen, um den Prothesenkern einfügen zu können. Danach nähert man die Prothesenhalterungen einander wieder, damit die Prothesenplatten den Prothesenkern in seiner Funktionsstellung einschließen, und das Instrument wird entfernt. Das bekannte Instrument ist als Zange ausgebildet, die gegenüber der Richtung der Prothesenhalterungen, die mit der Medianrichtung des Körpers übereinstimmen soll, abgewinkelt ist, um dem Einbringen des Prothesenkerns nicht im Wege zu sein. Dies hat verschiedene Nachteile. Insbesondere kann die Kraft zum Einbringen der Prothesenteile in den Zwischenwirbelraum nicht in Längsrichtung des Instruments aufgebracht werden. Dafür ist vielmehr ein besonderes Ansatzinstrument erforderlich.

Es ist auch ein Instrument zum Einbringen einer derartigen Prothese bekannt (DE-U-299 16 078), das von einem unteren Paar von Führungsstangen und einer oberen Führungsstange gebildet ist, die am hinteren Ende aneinander angelenkt sind und an ihren vorderen Enden Prothesenhalterungen tragen. Sie bilden eine Führungsbahn für ein Spreizelement. Wenn dieses mittels einer Zahnstange zwischen ihnen vorwärts getrieben wird, spreizt es die Stangenenden auseinander und schiebt gleichzeitig den Prothesenkern vor sich her, bis dieser die gewünschte Endstellung erreicht hat. Danach wird das Spreizelement zurückgezogen, um die Prothesenplatten dem Prothesenkern zu nähern. Dabei ist die Spreizbewegung mit der Einführung des Prothesenkerns zwangsläufig gekoppelt, so daß der Spreizvorgang nicht gesondert überwacht und nur schwer beobachtet werden kann.

Die Erfindung sucht ein Instrument zu schaffen, das eine von dem Einbringen des Prothesenkerns unabhängige Spreizung der Prothesenplatten erlaubt und dessen Längsrichtung beim Einbringen der Prothesen mit der Medianrichtung des Körpers übereinstimmt.

Die erfindungsgemäße Lösung liegt in den Merkmalen des Anspruchs 1.

Er betrifft ein Instrument zum Einsetzen einer Zwischenwirbelprothese, das zwei durch eine Parallelführung verbundene, voneinander weg spreizbare Prothesenhalterungen für ein Paar von Prothesenplatten aufweist. Die erste dieser Prothesenhalterungen ist fest an einem langgestreckten Instrumentenkörper übereinstimmend mit dessen Längsrichtung angeordnet. Die zweite Prothesenhalterung ist vermittelst der Parallelführung an dem Instrumentenkörper gehalten. Es ist ein Schräglenker vorgesehen, dessen erstes Ende an dem Instrumentenkörper in dessen Längsrichtung beweglich angelenkt ist. Sein zweites Ende ist an dem zweiten Prothesenhalter festachsig angelenkt. Sein erstes Ende ist mit einer in Längsrichtung des Instrumentenkörpers bewegbaren Betätigungseinrichtung verbunden. Wird die Betätigungseinrichtung in derjenigen Richtung bewegt, in welcher das erste Ende des Schräglenkers in Richtung zu dem Anlenkpunkt seines zweiten Endes bewegt wird, so richtet sich der Schräglenker auf und spreizt dadurch die zweite Prothesenhalterung vom Instrumentenkörper und der ersten Prothesenhalterung ab, und umgekehrt.

Der Schräglenker kann Teil einer Scheren-Parallelführung sein. Zweckmäßigerweise ist der Schräglenker paarig symmetrisch beiderseits des Instrumentenkörpers vorgesehen, um eine unsymmetrische und zum Verkanten neigende Kraftübertragung zu vermeiden.

Zweckmäßigerweise sind sämtliche den Instrumentenkörper und die zweite Prothesenhalterung verbindenden Teile außerhalb einer mittleren, in Längsrichtung des Instrumentenkörpers verlaufenden Durchtrittsöffnung angeordnet, deren Weite mindestens den Querabmessungen des zwischen die Prothesenplatten einzusetzenden Prothesenkerns und eines dafür vorgesehenen Prothesenkernhalters entspricht. Der Prothesenkern kann auf diese Weise leicht durch das Einsatzinstrument hindurch und durch dessen beidseitige Glieder geführt eingebracht werden. Damit der Operateur beim Einbringen des Prothesenkerns leicht diejenige Stellung des Prothesenkernhalters findet, in welcher der Prothesenkern die gewünschte Stellung zwischen den Prothesenplatten erreicht hat, sind das Einsetzinstrument und der Prothesenkernhalter zweckmäßigerweise mit zusammenwirkenden Anschlägen versehen, die diese Endstellung bestimmen.

Die Betätigungseinrichtung umfaßt zweckmäßigerweise eine Handhabe und eine Übersetzungseinrichtung. Die Übersetzungseinrichtung kann beispielsweise von einer Gewindespindel gebildet sein. Als zweckmäßiger hat es sich erwiesen, die Betätigungseinrichtung als einen Griffhebel auszubilden, der mit einem kürzeren Arbeitshebel verbunden ist, der die Übersetzungseinrichtung bildet. Er ist zweckmäßigerweise so angeordnet, daß er gleichzeitig die seitliche Bewegung des Griffhebels umwandelt in die in Längsrichtung des Instrumentenkörpers verlaufende Betätigungsrichtung.

Der Schräglenker kann mit seinem ersten Ende an einem am Instrumentenkörper in dessen Längsrichtung geführten Schlitten angelenkt sein. Statt dessen ist es auch möglich, daß er ein Glied eines Kniehebelpaars ist, wobei die Betätigungseinrichtung direkt oder indirekt am Kniepunkt des Hebelpaars angreift.

Die Erfindung wird im Folgenden näher unter Bezugnahme auf die Zeichnung erläutert, die ein vorteilhaftes Ausführungsbeispiel veranschaulicht. Es zeigen:
- Fig. 1: eine Gesamtansicht des Instruments,
- Fig. 2: eine perspektivische Ansicht des vorderen Abschnitts des Instruments von schräg oben,
- Fig. 3: eine Seitenansicht des vorderen Abschnitts,
- Fig. 4: eine Unteransicht des vorderen Abschnitts,

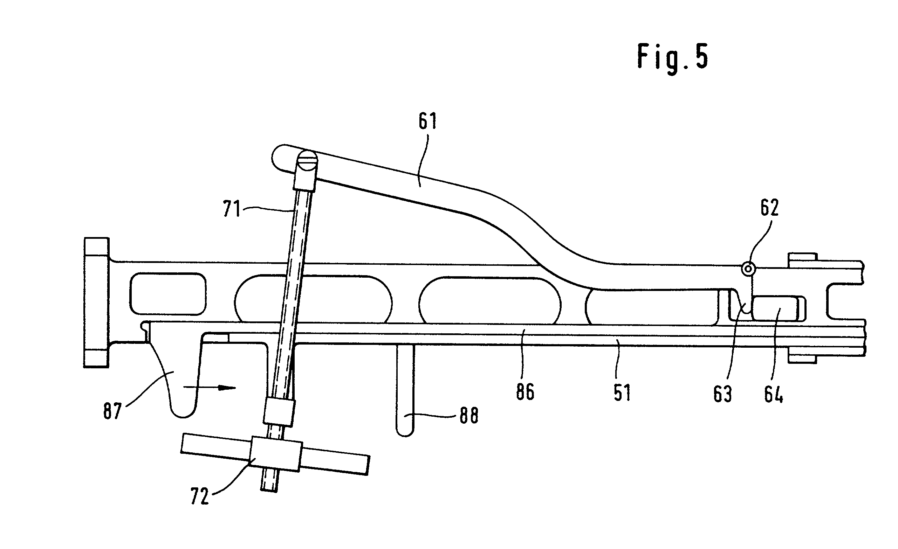

- Fig. 5: eine Unteransicht des hinteren Teils des Instruments,
- Fig. 6: ein Detail der Betätigungseinrichtung und
- Fig. 7: eine alternative Ausführung der Spreizeinrichtung.

Am vorderen Ende des Instrumentenkörpers 51 befinden sich zwei Halterungen 52 für Prothesenplatten 53. Die Prothesenhalterungen 52 sind gabelförmig und am Ende offen. Ihre seitlichen Schenkel bilden Führungen für den Rand der Prothesenplatten 53. Sie gestatten es, die Prothesenplatten unter Überwindung einer Reibkraft in Längsrichtung des Instruments in die Prothesenhalterungen 52 einzusetzen bzw. herausgleiten zu lassen. Am hinteren Ende besitzt der Prothesenkörper 51 eine Schlagplatte 54. Durch Schläge auf diese Platte können die Prothesenplatten 53, gehalten von den Prothesenhalterungen 52, zwischen zwei Wirbelkörper getrieben werden.

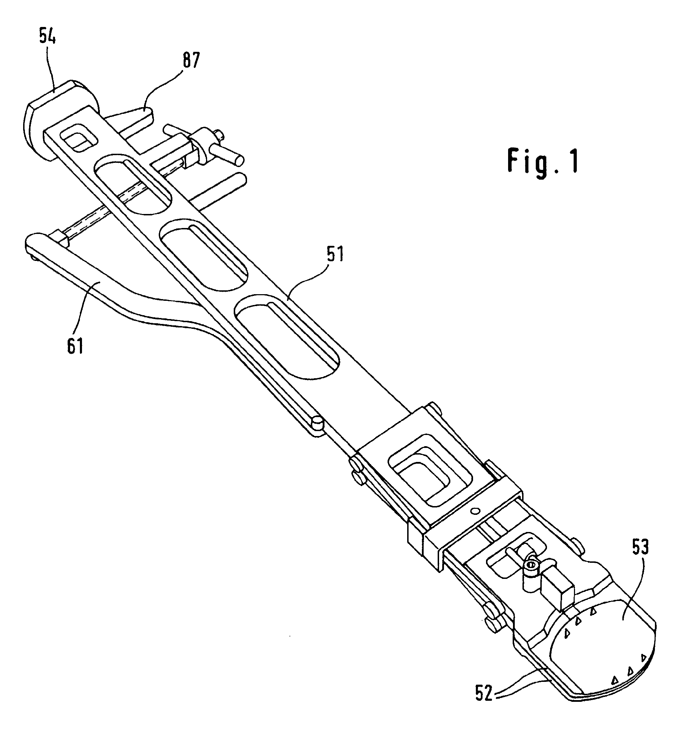

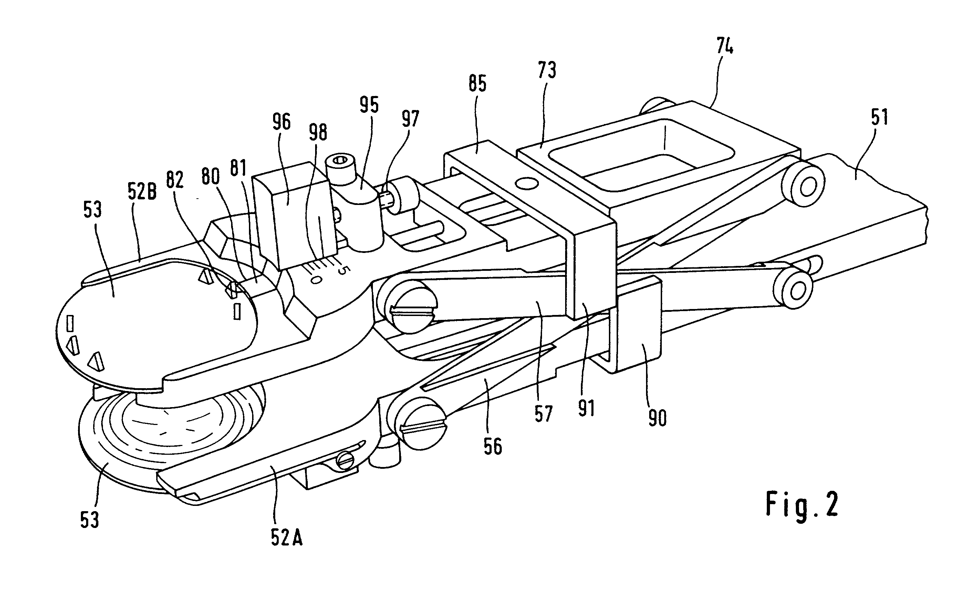

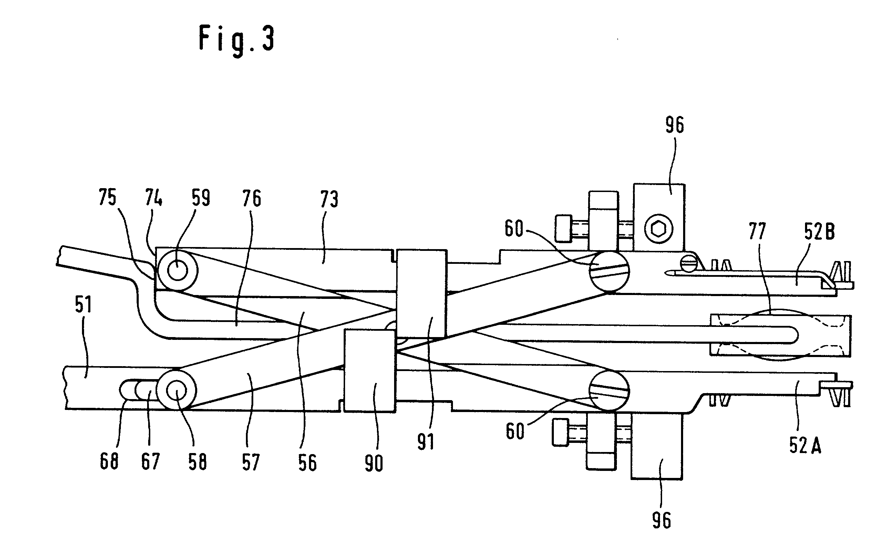

Die untere Prothesenhalterung 52A (Fig. 9 und 10) ist mit dem Instrumentenkörper 51 fest und im dargestellten Beispiel sogar einstückig verbunden. Die obere Prothesenhalterung 52B ist mittels einer Scherenanordnung, die aus Scherengliedern 56, 57 besteht, mit dem Instrumentenkörper 51 verbunden. Die Scherenanordnung 56, 57 ist so gestaltet, daß die obere Prothesenhalterung 52B sich ausschließlich lotrecht zur unteren Prothesenhalterung 52A und parallel zu dieser bewegen kann. Die Prothesenhalterungen können einander weitgehend angenähert werden (Fig. 1), um leichter in den Wirbelzwischenraum eingetrieben werden zu können. Sie können zusammen mit den benachbarten Wirbelkörpern auseinandergespreizt werden (Fig. 2 und 3), um Raum zu geben für die Einführung des Prothesenkerns 77 zwischen die Prothesenplatten 53. Danach werden sie einander wieder angenähert, um den Prothesenkern in der gewünschten Stellung festzuhalten. Das Instrument kann dann abgezogen werden.

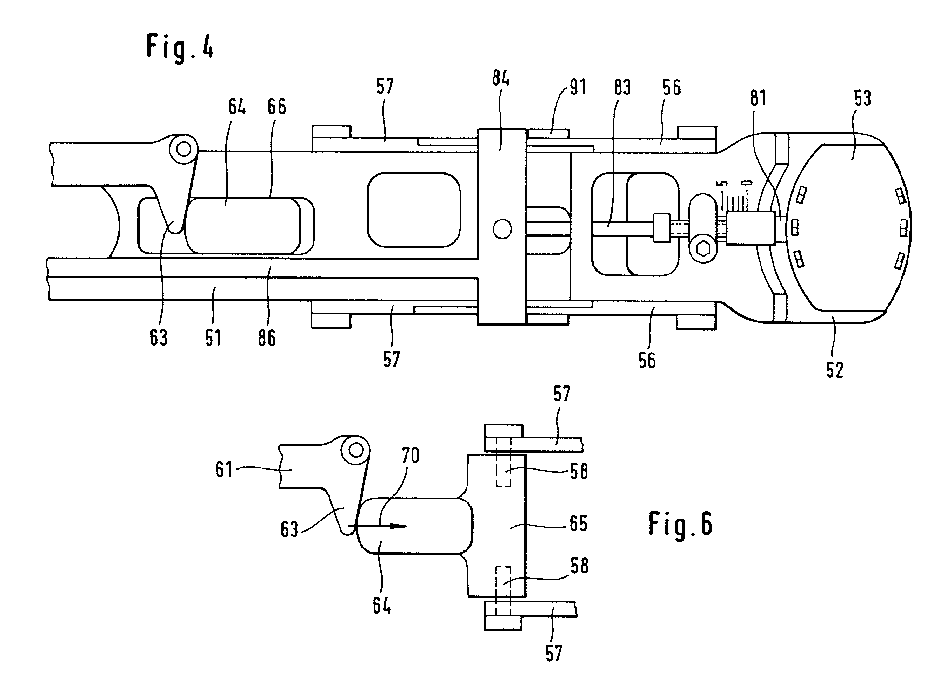

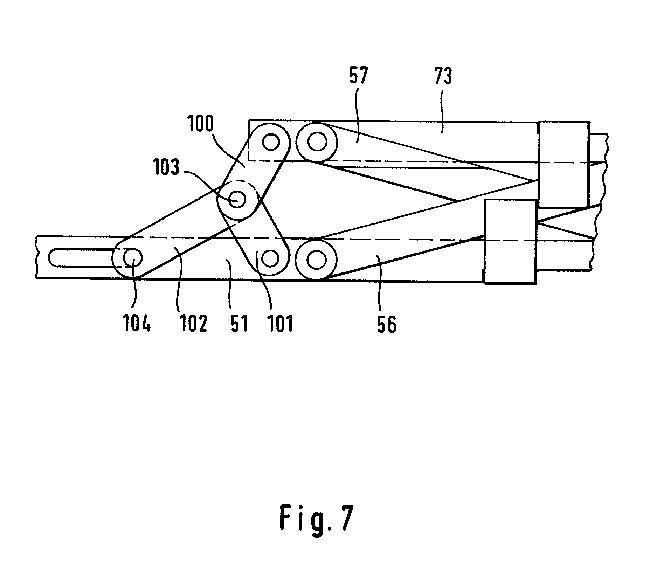

Die hinteren Bolzen 58, 59 der Scherenglieder 56, 57 gleiten in Langlöchern des Instrumentenkörpers 51 bzw. der Platte 73, die die obere Prothesenhalterung 52B nach hinten fortsetzt. Die Richtung der Langlöcher stimmt mit der Instrumentenlängsrichtung überein. Die vorderen Bolzen 60 der Scherenglieder 56, 57 sind starr mit den Prothesenhalterungen 52 verbunden. Um die Prothesenhalterungen zu spreizen, ist eine Einrichtung vorgesehen, die den hinteren Bolzen 58 des Scherenglieds 57 in Längsrichtung des Instruments verschiebt. Dafür ist der Griffhebel 61 vorgesehen, der am Instrumentenkörper um eine Achse 62 schwenkbar ist und einen Arbeitshebel 63 aufweist, der auf das hintere Ende eines Gleitsteins 64 wirkt, der Teil eines T-förmigen Schlittens 65 (Fig. 4 und 6) ist, an dessen Querhaupt die hinteren Bolzen 58 der beiderseits angeordneten Scherenglieder 57 angelenkt sind. Der Schlitten 65 ist in Längsrichtung des Werkzeugkörpers geführt. Man erkennt in Fig. 4, daß die parallelen Kanten des Gleitsteins 64 zwischen entsprechend parallelen Kanten 66 eines Ausschnitts im Instrumentenkörper geführt sind. In Fig. 3 erkennt man, daß die Enden 67 des Querhaupts in Langlöchern 68 geführt sind. Wird der Griffhebel 61 wie beim Zusammendrücken der Hebel einer Zange an den Instrumentenkörper herangezogen, so drückt sein Arbeitshebel 63 den Schlitten 64 in Pfeilrichtung 70 (Fig. 6). Dadurch wird das hintere Ende des Scherenglieds 57 nach vorne getrieben, wodurch die Prothesenhalterungen 52 auseinandergespreizt werden. Der Arbeitshebel 63, der Schlitten 65 und die Schräglenker 57 bilden somit eine Anordnung zum Verstellen des Abstands der Prothesenhalterungen 52. Es versteht sich, daß diese Anordnung auch durch andere Ausführungen ersetzt werden kann. Ferner erkennt man, daß die Spreizkraft nicht unbedingt über Teile der Scherenanordnung ausgeübt zu werden braucht.

Falls der Winkel zwischen dem Schräglenker 57 und der Längsrichtung des Instruments zu klein ist für die Ausübung einer großen Spreizkraft, kann für die Spreizung ein gesondertes Glied vorgesehen werden. Diese Alternative ist in Fig. 7 veranschaulicht. Die Platte 73, die an ihrem vorderen Ende die obere Prothesenhalterung 52 trägt, ist - wie in dem zuvor beschriebenen Ausführungsbeispiel - vermittelst einer Scherenanordnung 56,57 von dem Instrumentenkörper 51 getragen. Abweichend von jener Ausführung ist die Spreizeinrichtung unabhängig von der Scherenanordung verwirklicht. Die Lenker 100,101 bilden eine Kniehebelanordnung. Ein Ende dieser Lenker ist mit dem Instrumentenkörper 51 bzw. der Platte 73 verbunden. Ihr anderes Ende bildet das Knie 103, an dem das Ende eines Lenkers 102 eingreift, dessen anderes Ende 104 mit der Betätigungseinrichtung verbunden ist. Die Verbindung kann so ausgeführt sein, wie dies in Fig. 6 dargestellt ist. Die von der Scherenanordnung 56,57 unabhängige Spreizanordnung hat den Vorteil, daß die Winkel, unter denen die Lenker 100,101 und 102 beansprucht werden, ohne Rücksicht auf eine Parallelführungsfunktion lediglich im Hinblick auf eine günstige Kraftübertragung bemessen werden können.

Beim Spreizen der Prothesenhalterungen treten beträchtliche Kräfte auf. Deshalb ist der Handhebel 61 durch eine Gewindespindel 71 mit Knebelmutter 72 ergänzt, die den Vorgang erleichtert und es gestattet, das Instrument in der gespreizten Stellung zeitweilig festzusetzen.

In dieser Stellung bildet sich zwischen dem Instrumentenkörper 51 und der die obere Prothesenhalterung 52 nach hinten fortsetzenden Platte 53 einerseits sowie zwischen den seitlichen Scherenanordnungen 56, 57 andererseits ein kanalartiger Freiraum. Durch diesen Freiraum kann mittels eines Instruments 76 der Prothesenkern 77 zwischen die Prothesenplatten 52 geführt werden (Fig. 3). Das Instrument 76 weist einen Anschlag 75 auf, der sich an die Hinterkante 74 der Platte 73 anlegt, wenn der Prothesenkern 77 genau die vorgesehene Stellung zwischen den Prothesenplatten 52 erreicht hat.

Es wird nun eine Einrichtung zum Auswerfen der Prothesenplatten 53 aus den Prothesenhalterungen 52 bzw. zum Abdrücken des Instruments von den Prothesenhalterungen bzw. den angrenzenden Wirbeln beschrieben. Die Prothesenführungen 52 enthalten hinter dem Aufnahmeraum für die Prothesenplatten 53 eine Führungsnut 80, die in Instrumentenlängsrichtung und somit in der Gleitrichtung der Prothesenhalterungen 52 verläuft. Sie enthält einen Schieber 81, dessen vorderes Ende 82 am Rand der in der Prothesenhalterung befindlichen Prothesenplatte anschlägt und deswegen als Prothesenanschlag bezeichnet wird. Das hintere, in Fig. 2 und 3 nicht sichtbare Ende des Schiebers 81 ist mit einer ebenfalls in Instrumentenlängsrichtung geführten Stange 83 starr verbunden. Das hintere Ende der im Instrumentenkörper 51 gelagerten Stange 83 ist, wie Fig. 4 zeigt, an einem Anschlagelement 84 befestigt, dessen Natur später erläutert wird. Es ist ebenfalls in Längsrichtung des Instruments verschiebbar. Das Anschlagelement 84 ist wiederum starr mit einer Schubstange 86 verbunden, die im Instrumentenkörper 51 längs verschieblich gelagert ist und zu einer Handhabe 87 führt. Wenn der Operateur die Handhabe 87 in Pfeilrichtung nach vorne schiebt, werden die Schubstange 86, das Anschlagelement 84, die Stange 83 und der Schieber 81 nach vorne verschoben, um die Prothesenplatte 53 aus der Prothesenhalterung 52 herauszuschieben. Dabei kann sich die Hand des Operateurs an einem Zapfen 88 abstützen, der fest mit dem Instrumentenkörper 51 verbunden ist.

Unmittelbar wirkt sich die Bewegung der Handhabe 87 nur auf den Schieber 81 aus, der im unteren Teil des Instruments, nämlich im Instrumentenkörper, angeordnet ist. Damit sich die Schieber 81 beider Prothesenhalterungen synchron bewegen, ist eine Bewegungsübertragungseinrichtung vorgesehen. Die den Schieber 81 der oberen Prothesenhalterung steuernde Stange 83 ist an ihrem hinteren Ende mit einem Anschlagelement 85 fest verbunden, das ebenso wie das Anschlagelement 84 der unteren Prothesenhalterung in Instrumentenlängsrichtung beweglich geführt ist. Das untere Anschlagelement 84 weist beiderseits hochragende Anschlagschenkel 90 auf, die hinter und benachbart den Schenkeln 91 liegen, die von dem oberen Anschlagelement 85 beiderseits herunterragen. Wenn die Prothesenplatten 53 sich in ihrer hintersten Stellung in den Prothesenhalterungen 52 befinden und die Prothesenanschläge 82 sie berühren, liegen auch die einander benachbarten Stirnflächen der Anschlagschenkel 90, 91 aneinander an. Wenn nun durch Betätigung der Handhabe 87 das untere Anschlagelement 84 mit den Anschlagschenkeln 90 nach vorne geschoben wird, wird durch deren Zusammenwirken mit den Anschlagschenkeln 91 des oberen Anschlagelements auch der Schieber 81 der oberen Prothesenhalterung nach vorne geschoben. Die beiden Schieber 81 bewegen sich somit synchron. Da die zusammenwirkenden Anschlagflächen 90, 91 lotrecht zur Instrumentenlängsrichtung verlaufen, ist die synchrone Bewegung der Schieber 81 unabhängig von dem jeweiligen Abstand der Prothesenhalterungen voneinander gewährleistet.

Jeder Schieber 81 trägt einen starr mit ihm verbundenen Ansatz 95 sowie ein in Längsrichtung des Schiebers an diesem geführtes Klötzchen 96, das mit seiner Stirnfläche den Wirbelanschlag bildet. Wenn die Prothesenhalterungen mit den darin befindlichen Prothesenplatten 53 in den Zwischenraum zweier Wirbel eingetrieben werden, legen sich die Stirnflächen der Wirbelanschläge 96 schließlich an die ventralen Kanten der Wirbelkörper an. Durch den Abstand der Stirnflächen der Wirbelanschläge 96 von den Prothesenplatten wird somit die Tiefe bestimmt, in der die Prothesenplatten in den Wirbelzwischenraum gelangen. Durch Verstellen der Wirbelanschläge 96 an den Schiebern 81 kann diese Tiefe verändert werden. Dies geschieht mittels einer Gewindespindel 97, die in einer Gewindebohrung des Ansatzes 95 geführt ist und deren Ende drehbar, aber in Längsrichtung fest mit dem Wirbelanschlag 96 verbunden ist. Durch Verdrehen der Gewindespindel 97 kann somit der Operateur die Einsetztiefe der Prothesenplatten 53 im Verhältnis zur ventralen Kante der zugehörigen Wirbelkörper vorherbestimmen. Dabei hilft ihm eine Skala 98.

## Patentansprüche

1. Instrument zum Einsetzen einer Zwischenwirbelprothese, das durch eine Parallelführung (56,57) verbundene, voneinander wegspreizbare Prothesenhalterungen (52A) für ein Paar von Prothesenplatten (53B) aufweist, **dadurch gekennzeichnet, daß** die erste Prothesenhalterung (52A) fest an einem langgestreckten Instrumentenkörper (51) angeordnet ist und die andere durch die Parallelführung (56,57) mit dem Instrumentenkörper (51) verbunden ist und daß ein Schräglenker (57,100) vorgesehen ist, dessen erstes Ende (58,103) an dem Instrumentenkörper (51) in dessen Längsrichtung beweglich und dessen zweites Ende (60) an dem zweiten Prothesenhalter festachsig angelenkt ist, wobei dessen erstes Ende (58,103) mit einer in Längsrichtung des Instrumentenkörpers (51) bewegbaren Betätigungseinrichtung (61,63) verbunden ist.

2. Instrument nach Anspruch 1 **dadurch gekennzeichnet, daß** der Schräglenker (57,100) paarig symmetrisch beiderseits des Instrumentenkörpers vorgesehen ist.

3. Instrument nach Anspruch 1 oder 2 **dadurch gekennzeichnet, daß** der Schräglenker (57) Teil einer Scheren-Parallelfüh-rung (56,57) ist.

4. Instrumenten nach einem der Ansprüche 1 bis 3 **dadurch gekennzeichnet daß** alle den Instrumentenkörper (51) und die zweite Prothesenhalterung (52B) verbindenden Teile beiderseits eine mittlere, in Längsrichtung des Instrumentenkörpers (51) kanalartig verlaufende Durchtrittsöffnung begrenzen, deren Weite mindestens den Querabmessungen eines zwischen die Prothesenplatten (53) einzusetzenden Prothesenkerns (77) und eines dafür vorgesehenen Prothesenkernhalters (76) entspricht.

5. Instrument nach Anspruch 4 **dadurch gekennzeichnet, daß** das Instrument und der Prothesenkernhalter (76) zusammenwirkende Anschläge (74,75) zum Bestimmen der Endstellung des Prothesenkernhalters (76) aufweisen.

6. Instrument nach einem der Ansprüche 1 bis 5, **dadurch gekennzeichnet, daß** die Betätigungseinrichtung eine Handhabe (61) und eine Übersetzungseinrichtung (63) umfasst.

7. Instrument nach Anspruch 6, **dadurch gekennzeichnet, daß** die Handhabe von einem Griffhebel (61) und die Übersetzungseinrichtung von einem mit dem Griffhebel (61) verbundenen, kürzeren Arbeitshebel (63) gebildet ist.

8. Instrument nach einem der Ansprüche 1 bis 7, **dadurch gekennzeichnet, daß** das erste Ende (58) an einem am Instrumentenkörper in dessen Längsrichtung geführten Schlitten (65) angelenkt ist.

9. Instrument nach einem der Ansprüche 1 bis 7, **dadurch gekennzeichnet, daß** der Schräglenker (100) ein Glied eines Kniehebelpaars (100,101) ist.
